(19) Europäisches Patentamt

European Patent Office

Office européen des brevets

(11) Publication number: **0 416 890 A1**

# EUROPEAN PATENT APPLICATION

(21) Application number: 90309705.3

(22) Date of filing: 05.09.90

(51) Int. Cl.⁵: **C12N 9/64**, C12N 11/10

(30) Priority: 05.09.89 US 403516

(43) Date of publication of application:
**13.03.91 Bulletin 91/11**

(84) Designated Contracting States:
**AT BE CH DE DK ES FR GB GR IT LI LU NL SE**

(71) Applicant: **ELI LILLY AND COMPANY**
**Lilly Corporate Center**
**Indianapolis Indiana 46285(US)**

(72) Inventor: **Yan, Sau-Chi Betty**
**8131 Menlo Court East Drive**
**Indianapolis, Indiana 46240(US)**

(74) Representative: **Hudson, Christopher Mark et al**
**Erl Wood Manor**
**Windlesham Surrey GU20 6PH(GB)**

(54) Method for activating protein C.

(57) The activation of protein C molecules, whether derived from plasma or produced by recombinant DNA technology, can be effected by contacting the protein C molecules with immobilized thrombin. The present method is more efficient than using a thrombin/thrombomodulin complex and removes the problem of thrombin self-degradation.

EP 0 416 890 A1

# METHOD FOR ACTIVATING PROTEIN C

The present invention provides a novel method for activating protein C. The method provides a simple and efficient means for converting protein C from the zymogen form into activated protein C. Prior art methods for activating protein C involved treatment of the inactive zymogen form of protein C with high levels of thrombin in solution, or thrombin and thrombomodulin together, or other expensive enzymes for activation. The present invention provides a method for activating protein C using immobilized thrombin, thereby circumventing the need for thrombomodulin and resulting in an activated protein C molecule which can be easily purified away from the immobilized thrombin complex.

## The Role of Protein C in the Regulation of Blood Coagulation

Protein C, a vitamin K dependent plasma protein, is of major physiological importance in the control of hemostasis. Protein C is synthesized as an inactive molecule, herein called nascent protein C. Nascent protein C undergoes complex processing, giving rise to a number of different inactive molecules as is more fully described below. Inactive, secreted forms of protein C are referred to herein as zymogen protein C. Activation of protein C occurs in the blood by a reaction involving a thrombomodulin-thrombin complex. Activated protein C, together with its cofactor protein S, is an anticoagulant of important physiological significance. Activated protein C can prevent intravascular thrombosis and control the extension of existing clots. The mechanism of action of the activated form of protein C and the mechanism of activation of the inactive zymogen into the active protease have been clarified in recent years (for review, see J. E. Gardiner and J. H. Griffin, Progress in Hematology, Vol. XIII, pp. 265-278, ed. Elmer B. Brown, Grune and Stratton, Inc., 1983).

The activation of protein C involves thrombin, the final serine protease in the coagulation cascade, and an endothelial cell membrane-associated glycoprotein called thrombomodulin. Thrombomodulin forms a tight, stoichiometric complex with thrombin. Thrombomodulin, when complexed with thrombin, totally changes the functional properties of thrombin. Thrombin normally clots fibrinogen, activates platelets, and converts clotting cofactors V and VIII to their activated forms, Va and VIIIa. Finally, thrombin activates protein C, but only very slowly and inefficiently. In contrast, thrombin complexed with thrombomodulin does not clot fibrinogen, activate platelets, or convert clotting factors V and VIII to their activated counterparts Va and VIIIa, but does become a very efficient activator of protein C. The rate constant of protein C activation by thrombomodulin-thrombin is over 1,000 fold higher than the rate constant for thrombin alone.

To understand how activated protein C down-regulates blood coagulation, the following brief description of the coagulation enzyme system is provided. The coagulation system is best looked at as a chain reaction involving the sequential activation of zymogens into active serine proteases. This chain reaction eventually produces the enzyme thrombin, which through limited proteolysis converts plasma fibrinogen into the insoluble gel fibrin. Two key events in the coagulation cascade are the conversion of clotting factor X to Xa by clotting factor IXa and the conversion of prothrombin into thrombin by clotting factor Xa. Both of these reactions occur on cell surfaces, most notably the platelet surface, and both reactions require cofactors. The major cofactors, factors V and VIII, in the system circulate as relatively inactive precursors, but when the first few molecules of thrombin are formed, thrombin loops back and activates the cofactors through limited proteolysis. The activated cofactors, Va and VIIIa, accelerate both the conversion of prothrombin into thrombin and also the conversion of factor X to factor Xa by approximately five orders of magnitude. Activated protein C preferentially acts on, to proteolytically degrade, hydrolyze, and irreversibly destroy clotting cofactors Va and VIIIa, the activated forms of the inactive clotting factors V and VIII. Clotting factors V and VIII, in contrast, are very poor substrates for activated protein C.

An important cofactor for activated protein C is protein S, another vitamin K-dependent plasma protein. Protein S substantially increases activated protein C-mediated hydrolysis of factors Va and VIIIa 25 fold.

## Protein C as a Therapeutic Agent

Protein C is recognized as a valuable therapeutic agent (see, for example, European Patent Publication No. 0215548 and U.S. Patent No. 4,775,624, incorporated herein by reference). Activated protein C is a novel antithrombotic agent with a wider therapeutic index than available anticoagulants, such as heparin and the oral hydroxycoumarin type anticoagulants. Neither zymogen protein C nor activated protein C is effective until thrombin is generated, because thrombin is needed to convert clotting factors V to Va and VIII to VIIIa; the activated forms of these two cofactors are the preferred substrate for activated protein C. Thrombin is also required to activate zymogen protein C, for without the thrombomodulin-thrombin complex, the protein C zymogen is not converted into its active counterpart.

Activated protein C is an on-demand anti-coagulant, because activated protein C works by inactivating cofactors Va and VIIIa. Because thrombin is required to convert factors V and VIII to their activated counterparts Va and VIIIa, protein C only acts as an anticoagulant after thrombin is generated. Conventional anticoagulants, in contrast to activated protein C, maintain a constant anticoagulant state throughout the circulation for as long as they are given to the patient, thereby substantially increasing the risk of bleeding complications over that for protein C or activated protein C. Activated protein C is therefore an on-demand anticoagulant of wide clinical utility for use as an alternative to heparin and the hydroxycoumarins.

In some disease states, such as hereditary protein C deficiency, protein C zymogen is of great therapeutic importance. In congenital homozygous protein C deficiency, affected individuals die in early childhood from purpura fulminans, an often lethal form of disseminated intravascular coagulation. In heterozygous protein C deficiency, affected individuals suffer severe, recurrent thromboembolic episodes. It is well established clinically that plasma protein concentrates designed to treat hemophilia B or factor IX deficiency, which contain protein C as an impurity, are effective in the prevention and treatment of intravascular clotting in heterozygous protein C deficiency. Protein C levels have also been noted to be abnormally low in thrombotic states such as disseminated intravascular coagulation and in disease states predisposing to thrombosis, such as major trauma, major surgery, and cancer.

Although the zymogen forms of protein C are quite useful for therapeutic purposes, some disease states can be treated much more effectively by delivering the activated form of protein C to the patient. For instance, in disease states such as myocardial infarction or deep vein thrombosis (especially as occurs after surgery on the lower extremities), patients have normal levels of protein C zymogen yet not enough activated protein C to prevent the generation of thrombi or to support the removal of existing thrombi. The inability to generate sufficient amounts of activated protein C may arise from inadequate thrombomodulin levels, but, whatever the cause, effective treatment of these disease states requires the administration of activated protein C and not the zymogen. The present invention provides a novel method for activating protein C using immobilized thrombin, rather than a thrombin/thrombomodulin complex.

## The Synthesis and Activation of Human Protein C

Nascent protein C can be depicted schematically, as shown below.

```
|1        42|43      197|198  199|200 211|212        461|
|pre-pro  |   LC     |   KR   |  AP  |    AHC      |

                                    <———————HC———————>
```

pre-pro - amino acid residues 1-42 of nascent human protein C encode the signal peptide and propeptide of human protein C, important for directing secretion and $\gamma$-carboxylation of protein C.

LC - amino acid residues 43-197 of nascent protein C, once post-translationally modified, constitute the light chain (LC) of both the two-chain zymogen (formed from one-chain zymogen by removal of the KR dipeptide, as discussed below) and activated forms of protein C.

KR - amino acid residues 198-199 of nascent human protein C; these residues are believed to be removed (on the basis of homology with bovine protein C), probably by a two-step process comprising a first cleavage (either between residues 197-198 or 199-200) followed by carboxypeptidase or aminopeptidase action, to form two-chain protein C.

AP - amino acid residues 200-211 of nascent protein C constitute the activation peptide, which is removed from the zymogen forms of protein C to obtain activated protein C.

AHC - amino acid residues 212-461 of nascent protein C, once post-translationally modified, constitute the activated heavy chain (AHC) of active protein C.

HC - the heavy chain of the two chain form of protein C zymogen, once post-translationally modified, which constitutes amino acid residues 200-461, the AP and AHC.

Human protein C zymogen is a serine protease precursor synthesized in the liver and present in the blood. For expression of complete biological activity, protein C requires post-translational modifications for which vitamin K is needed. The mature, two-chain, disulfide-linked, protein C zymogen arises from a single-chain precursor by limited proteolysis. This limited proteolysis is believed to include cleavage and removal of a pre-pro peptide consisting of amino acid residues 1-42 during intracellular processing and secretion of the nascent polypeptide from the cell and removal of amino acid residues 198 and 199 to form the two chains observed in the zymogen. The activation of the zymogen into the active serine protease involves the proteolytic cleavage of an ARG-LEU peptide bond (residues 211 and 212). This latter cleavage releases a dodecapeptide (residues 200-211) that constitutes the amino-terminus of the larger (heavy) chain of the two-chain zymogen molecule. Protein C is significantly glycosylated; the mature enzyme contains ~23% carbohydrate. Protein C also contains a number of unusual amino acids, including γ-carboxyglutamic acid and β-hydroxyaspartic acid (erythro-L-β-hydroxy aspartate). γ-carboxyglutamic acid (gla) is produced by γ-glutamyl carboxylation from glutamic acid residues with the aid of a hepatic microsomal carboxylase which requires vitamin K as a cofactor.

The activation of human protein C can also be represented schematically and is shown below. Those skilled in the art recognize that the order of the steps shown in the schematic do not necessarily reflect the in vivo pathway.

```
                              pre-pro-LC-KR-AP-AHC        nascent protein C

                                          |
        post-translational modification,  |
        i.e., γ-carboxylation of specific |
        glutamic acid residues, β-        |
        hydroxylation of an aspartic      |
        acid residue, and glycosylation   |
                                          ↓


        secretion, the removal of         |
        residues 1-42, which may          |
        involve more than one             |
        proteolytic cleavage              |
                                          ↓


                              LC-KR-AP-AHC        one-chain zymogen

                                          |
        removal of residues 198-199,      |
        about 90% of the zymogen protein  |
        C found in human blood is the     |
        two chain form (S-S= disulfide    |
        bond)                             |
                                          ↓

                              LC
                               |
                              S-S            two-chain zymogen
                               |
                              AHC-AP

        activation by                 |
        thrombin-thrombomodulin       |
                                      ↓

                              LC
                               |
                              S-S            activated protein C
                               |
                              AHC
```

The present invention provides a novel method for activating protein C zymogen using immobilized thrombin.

For purposes of the present invention, as disclosed and claimed herein, the following terms are as defined below.

Amino acid residues in proteins or peptides described herein as abbreviated as follows:

EP 0 416 890 A1

| Three-Letter Abbreviation | Amino Acid Residue | One-Letter Abbreviation |
|---|---|---|
| PHE | Phenylalanine | F |
| LEU | Leucine | L |
| ILE | Isoleucine | I |
| MET | Methionine | M |
| VAL | Valine | V |
| SER | Serine | S |
| PRO | Proline | P |
| THR | Threonine | T |
| ALA | Alanine | A |
| TYR | Tyrosine | Y |
| HIS | Histidine | H |
| GLN | Glutamine | Q |
| ASN | Asparagine | N |
| LYS | Lysine | K |
| ASP | Aspartic Acid | D |
| GLU | Glutamic Acid | E |
| CYS | Cysteine | C |
| TRP | Tryptophan | W |
| ARG | Arginine | R |
| GLY | Glycine | G |

Enh or enhancer - the enhancer of BK virus.

$\gamma$-carboxylation - a reaction which adds a carboxyl group to glutamic acids at the $\gamma$-carbon.

$\gamma$-carboxylated protein - a protein in which some glutamic acids residues have undergone $\gamma$-carboxylation.

Nascent protein - the polypeptide produced upon translation of a mRNA transcript, prior to any post-translational modifications. However, post- translational modifications such as $\gamma$-carboxylation of glutamic acid residues and hydroxylation of aspartic acid residues may occur before a protein is fully translated from an mRNA transcript.

Protein C activity - any property of human protein C responsible for proteolytic, amidolytic, esterolytic, and biological (anticoagulant or profibrinolytic) activities. Methods for testing for protein anticoagulant activity are well known in the art, i.e., see Grinnell et al ., 1987, Biotechnology 5:1189.

Zymogen - an enzymatically inactive precursor of a proteolytic enzyme. Protein C zymogen, as used herein, refers to secreted, inactive forms, whether one chain or two chain, of protein C.

The present invention provides a novel method for activating protein C, said method comprising the steps of:

a) contacting inactive protein C with immobilized thrombin, and

b) purifying the activated protein C away from the immobilized thrombin and other contaminants. Although several methods for producing inactive human protein C zymogen and inactive nascent human protein C have been described (see European Patent publication 215548 and U.S. Patent No. 4,775,624, the teachings of which are herein incorporated by reference), the disclosures do not provide for methods for the activation of protein C using immobilized thrombin. Instead, the protein C zymogens produced heretofor have been treated with substances such as $\alpha$-thrombin in solution, trypsin, Russell's viper venom factor X activator, or a mixture of thrombin and thrombomodulin to obtain activated protein C. All of these activation methods introduce inefficiency, risk of contamination, and higher costs to the recombinant production of activated human protein C. Some of the activation reactions must be closely monitored so that the proteolysis stops after the proteolytic cleavage of the activation peptide -- otherwise, the activated protein C produced is cleaved and rendered inactive. In addition, $\alpha$-thrombin tends to self-digest when maintained in solution, therefore necessitating a continual addition of $\alpha$-thrombin to the reaction mixture. The present method of activating protein C using immobilized thrombin removes the problem of thrombin self-digestion, as well as the need for thrombomodulin.

The method of the present invention can be used not only for the activation of human protein C zymogens formed by recombinant DNA technology, but also for the activation of naturally occurring human protein C isolated from blood plasma. Kisiel, W., 1979, J. Clin. Invest. 64 :761-769, the teaching of which is herein incorporated by reference, discloses one such method for the isolation of protein C from plasma.

6

Furthermore, the method of the present invention may also be used to activate protein C derivatives, such as those described by Bang et al ., European Patent Publication No. EP-O-323 149. Recombinant human protein C molecules which comprise novel glycosylation patterns may also be activated using the novel immobilized thrombin method of the present invention.

In general, the method of the present invention is practiced by immobilizing thrombin to any support resin. The type of thrombin used is dependent upon a number of factors, most particularly expense and safety. Bovine thrombin is generally preferred because it is less likely to be contaminated with human virus, although thrombin from human, horse, mouse or rat is also commercially available. Several classes of thrombin, such as $\alpha$-thrombin, $\beta$-thrombin and $\delta$-thrombin are also available and are useful in the present invention, although $\alpha$-thrombin is preferred.

Many different types of solid supports may be used to immobilize the thrombin used to activate protein C, although sepharose and agarose are preferred. An agarose affinity support with N-hydroxysuccinimide (NHS) esters as cross-linking reagents is most preferred. Thrombin contains lysine residues, the epsilon amino group of which is linked to the column in a non-amino buffer such as HEPES. The HEPES buffer is made at a pH of about 7.6 so as to maintain the stability of the thrombin and so that the lysine residues will become sufficiently deprotonated. The carbon atom found in the ester undergoes nucleophilic attack by the deprotonated lysine, thereby allowing replacement of the ester with the lysine residue on the thrombin. This reaction is very mild and the thrombin remains stable.

Once the thrombin is bound to the support, the immobilized thrombin can be equilibrated with the appropriate buffer and the protein C can be added for activation. The immobilized thrombin can be contacted with the protein C in a batch reaction, or the protein C can be passed though a column containing the immobilized thrombin. The protein C can be passed through one long column or reirculated through a short column and assayed for activation at any given point in the reaction scheme. The immobilized thrombin method is more efficient than the prior soluable thrombin method because immobilized thrombin does not degrade as readily as thrombin in solution. Immobilized thrombin columns are reuseable, often for as many as fifteen loadings. The immobilized thrombin method is more efficient than the prior immobilized thrombin/thrombomodulin method because the thrombomodulin used in the thrombin/thrombomodulin method is not covalently linked to the immobilized support and tends to contaminant the final eluate of activated protein C. Such cross-contamination is not significant (less than one part per million) when using immobilized thrombin alone.

The activity of activated protein C can be monitored by a variety of methods, although the most common are the factor Xa-one stage clotting assay or an Activated Partial Thromboplastin Time (APTT) clotting assay, both of which are described in Grinnell et al ., 1987, Biotechnology 5 :1189-1192, the entire teaching of which is herein incorporated by reference. Activation methods are also disclosed in Bang et al ., U.S. Patent No. 4,775,624, issued October 4, 1988, the entire teaching of which is herein incorporated by reference.

The first step in practicing the method of the present invention is dissolving about 50 mg of bovine $\alpha$-thrombin in about 25 ml of 50 mM HEPES buffer, pH 7.6. A 25 ml bottle of AffigelTM 10 beads are then washed with about 500 ml of very cold, highly purified water. After draining the excess water from the beads, the wet beads are added to the thrombin solution and gently mixed for one to two hours on a rotating device at 4°C. To block unreacted sites on the Affi-Gel-10TM resin, 100 $\mu$l of 1 M glycine, pH 8.0, is added to the solution and the rotation is continued for one to sixteen hours at 4°C.

The thrombin-beads (T-beads) are separated into two sterile 50 ml polystyrene tubes and centrifuged at half speed or less for 30 to 60 seconds. The tubes are placed on ice and the T-beads are allowed to settle for one to two minutes, then the supernatants are removed and discarded. About 12.5 ml of T-beads are left in each tube.

About 35 ml of 20 mM Tris-HCl, pH 7.4, 200 mM NaCl wash buffer is added to each tube, then the tubes are gently inverted until all of the T-beads are resuspended. The tubes are centrifuged as before, then placed on ice to complete settling. The supernatants are drawn off and discarded. This washing procedure is repeated 10 to 15 times, then the Amidolytic Activity of the last supernatant is checked to detect unbound or leached thrombin. If the change of optical density at 405 nm is less than 0.002/minute, then the T-beads are ready for use. If the OD is too high, the washing procedure must be repeated until the proper level is obtained.

Once the supernatant displays the proper level of purity, a 25% suspension of T-beads is prepared by adding 3 ml of the 20 mM Tris-HCl, pH 7.4, 200 mM NaCl wash buffer per milliliter of centrifuged T-beads. The T-beads can be stored for an extended period of time, if kept sterile at 4°C in the wash buffer.

The T-bead suspension is tested for the ability to activate protein C by a trial activation in a 1.5 ml polypropylene microcentrifuge tube at 37°C for 2 to 3 hours. The functional activity of the protein C can be assayed by both the S-2238 amidolytic procedure and the anticoagulant activity procedure disclosed by Grinnell et al ., 1987, Biotechnology 5 :1189-1192. The microcentrifuge tube containing the protein C immobilized thrombin is allowed to rotate for 30 to 60 minute intervals until the functional activity of the protein C reaches a plateau.

The invention is further illustrated by the following exemplification.

Example 1

Immobilization of Bovine α-Thrombin

One bottle (approximately 50 mg) of pyrogen-free, highly purified bovine thrombin (available from Miles Laboratories, Inc., 1121 Myrtle, Box 2000, Elkhart, Indiana 46515 or ICN Pharmaceuticals, Inc., 26201 Miles Road, Cleveland, Ohio, 44128) was dissolved in 25 ml of 50 mM HEPES buffer, pH 7.6. A 25 ml bottle of Affi-Gel™-10 beads (Bio-Rad Laboratories, P.0. Box 708, 220 Maple Avenue, Rockville Centre, New York 11571) was washed in a 150 ml glass frit funnel with about 300 to 500 ml of very cold, highly purified water. The beads were reslurried after each addition of water and were never allowed to go dry. The beads are very fragile and are easily disrupted.

The Thrombin solution was transferred into a sterile, 50 ml polystyrene centrifuge tube and the washed beads were added. The tube was capped and the contents gently mixed by rotation at 4° C for 1 to 2 hours on a rotating mixing apparatus. Next, 100 $\mu$l of 1M glycine solution, pH 8.0, was added to block unreacted sites on the Affi-Gel-10™ resin. The tube was recapped and rotated for between one and sixteen hours at 4° C. The Thrombin-beads (T-beads) were then divided between two separate 50 ml polystyrene centrifuge tubes, which were placed in an ice bath for further cooling. The tubes were centrifuged at one-half speed or less for 60 seconds in a bench top model Clinical Centrifuge (about 1000-2000 rpm). The tubes were carefully transferred to an ice bath and settling was allowed to occur for an additional one to two minutes. The supernatant was then removed from each tube with a sterile pipette, leaving approximately 12.5 ml of T-Beads in each tube.

Approximately 35 ml of T-Bead Wash Buffer (20 mM Tris-HCl (pH 7.4), 200 mM NaCl) was added to each tube, the tubes were tightly capped and gently inverted by hand until the T-Beads were resuspended. The tubes were centrifuged for 60 seconds at one-half speed, then placed in an ice bath for one to two minutes before the supernatants were drawn off with a sterile pipette. This washing procedure was repeated approximately 10 to 15 times to remove contaminating endotoxins, glycine and thrombin.

The supernatant was then checked using the S-2238 Amidolytic Assay. A bottle (25 mg) of Helena Laboratories S-2238 substrate was dissolved in 18 ml of 20 mM Tris-HCl, 150 mM NaCl, pH 7.4 which was sterile filtered through a 0.2 mm Acrodisc filter. A Dilution Buffer was next produced which is composed of 20 mM Tris-HCl (pH 7.4), 150 mM NaCl, 3 mM CaCl$_2$ and 1 mg/ml Bovine Serum Albumen (BSA). To check the T-Beads for leaching thrombin, the following protocol is performed. In a disposable microcuvette, 100 $\mu$l of the T-Bead supernatant is mixed with 600 $\mu$l of Dilution Buffer and 300 $\mu$l of the S-2238 Substrate solution. The cuvette is covered with parafilm then inverted to mix. The optical density at 405 nm was read for 4 minutes. If the $\Delta$O.D./minute is 0.002 or less, the T-Beads are sufficiently clean. If the $\Delta$O.D./minute is greater than 0.002, then the washing protocol must be repeated until the T-Beads are sufficiently free of leached thrombin.

Once the T-Beads were clean, the graduations on the side of the centrifuge tube were used to gauge the relative volume of the T-Beads. Three volumes of T-Bead Wash Buffer were then added to effect a dilution of 1 to 4 (25%). The T-Beads may be stored and held at 4° C for an extended period of time in this solution, if kept sterile.

To test for the ability of the T-Beads to activate protein C, 400 $\mu$l of the resuspended T-Beads were transferred to a 1.5 ml polypropylene micro-centrifuge tube, centrifuged at one-half speed for 60 seconds, then the supernatant was carefully removed. A 500 $\mu$l sample of high quality non-activated recombinant human protein C (1 mg/ml) was added to the T-Beads along with 10 $\mu$l of 0.2 M EDTA pH 7.4 (to remove Calcium). The tube was capped, gently inverted several times then centrifuged as before. A 100 $\mu$l sample was removed from the supernatant and mixed with 900 $\mu$l of T-Bead Dilution Buffer, then the O.D.$_{280}$ nm was read to assure that the sample was 1 mg/ml in recombinant Human Protein C (rHPC).

The reaction tube was recapped and gently rotated at 37° C for 2 to 4 hours. At the 30 minute, 1 hour, 2 hour and 4 hour time points, the tube was centrifuged and a 10 μl sample was removed. This 10 μl sample was diluted to give a final concentration of 10 μg/ml rHPC. This diluted sample in 50 μl was then added to 650 μl of Activity Assay Dilution Buffer and 300 μl S-2238 Substrate, then gently mixed by inversion. The O.D.405 nm was read for 4 minutes. Any change in O.D greater than 0.1/minute indicates that the rHPC is fully active.

Example 2

Activation of rHPC

Recombinant HPC was prepared in substantial accordance with the teaching of Bang et al ., U.S. Patent No. 4,775,624 and Grinnell et al ., 1987, Biotechnology 5 :1189-1194. One hundred ml of Pharmacia Fast Flow Q (FFQ) resin was properly prepared as recommended by the manufacturer. The FFQ resin was then equilibrated with a buffer solution containing 20 mM Tris, 0.15 M NaCl, 2 mM EDTA, 2 mM benzamidine (pH 7.4). EDTA and benzamidine were added to the cell culture supernatants to a final volume of 4 mM and 5 mM respectively. The culture media was passed through the FFQ column (3 x 16 cm) at a linear flow rate of 20 cm.h⁻¹. The column was washed first with 300 ml (3 column volumes) of a solution containing 20 mM Tris, 0.15 M NaCl, 2 mM EDTA, 2 mM benzamidine (pH 7.4), then 300 ml (3 column volumes) solution containing 20 mM Tris, 0.15 M NaCl, 10 mM caCl₂ and 2 mM benzamidine (pH 7.4). This second eluate was then concentrated using classical anion exchange procedures.

The purified rHPC-containing solution was filtered through sterile 0.2 μm Acrodisc filters into a polypropylene container. The concentration of rHPC is in a range of 1-10 mg/ml in 20 mM Tris, pH 7.4, 0.15 M NaCl and in the absence of caCl₂. (Pre-Activation Buffer). The T-Beads produced in Example 1 were washed with T-Bead Wash Buffer, then centrifuged and the supernatant was discarded. About 5 to 7 ml of T-Beads are sufficient to activate up to 45 ml of purified rHPC. The rHPC in the Pre-Activation Buffer was added to the T-Beads, then EDTA was added to a final concentration of 0.5 mM. The tube was capped and gently rotated at 37°C. Aliquots were periodically removed and the Amidolytic Activity was assayed in substantial accordance with the teaching of Example 1. Protein C treated by this method was found to be 100% activated within 2 hours.

Those skilled in the art will recognize, or be able to ascertain, using no more than routine experimentation, numerous equivalents to the specific substances and procedures described herein. Such equivalents are considered to be within the scope of this invention, and are covered by the following claims.

**Claims**

1. A method for activating protein C, said method comprising the steps of:
   a) contacting inactive protein C with immobilized thrombin, and
   b) purifying the activated protein C away from the immobilized thrombin and other contaminants.
2. The method of Claim 1 wherein the thrombin is selected from the group consisting of bovine thrombin, horse thrombin, human thrombin, mouse thrombin and rat thrombin.
3. The method of Claim 1 wherein the thrombin is selected from the group consisting of α-thrombin, β-thrombin and γ-thrombin.
4. The method of Claim 1 wherein the thrombin is immobilized on agarose.
5. The method of Claim 1 wherein the thrombin is immobilized on sepharose.
6. The method of Claim 1 wherein the protein C is selected from the group consisting of bovine protein C, human protein C and human protein C derivatives.
7. The method of Claim 6 wherein the protein C is human protein C.
8. The method of Claim 2 wherein the thrombin is bovine α-thrombin.
9. The method of Claim B wherein the bovine α-thrombin is immobilized on agarose.
10. The method of Claim 9 wherein the protein C is human protein C.

European
Patent Office

EUROPEAN SEARCH
REPORT

Application Number

EP 90 30 9705

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BLOOD vol. 59, no. 5, 05 May 1982, pages 1067 - 1072; MARTAR,R.A.et al: "Mechanism of action of human activated protein C, a thrombin-dependant anticoagulant enzyme" * pages 1067 - 1069 * | 1-3,5-8, 10 | C 12 N 9/64 C 12 N 11/10 |
| Y | idem | 4,9 | |
| Y | WO-A-8 900 205 (AMERICAN NATIONAL RED CROSS) * pages 8 - 11 * | 4,9 | |
| X,P | WO-A-8 912 685 (INTEGRATED GENETICS) * pages 31 - 32 * | 1-3,5-8, 10 | |

TECHNICAL FIELDS
SEARCHED (Int. Cl.5)

C 12 N

The present search report has been drawn up for all claims

| Place of search | Date of completion of search | Examiner |
|---|---|---|
| The Hague | 09 November 90 | FERNANDEZ Y BRANAS F |